# EUROPEAN PATENT SPECIFICATION

(11) **EP 2 956 062 B1**
(45) Date of publication and mention of the grant of the patent: **02.07.2025**
(21) Application number: 14702636.3
(22) Date of filing: 05.02.2014
(51) Int. Cl.: A61B 5/11, G06T 7/20, A61B 5/113, G06T 7/00, A61B 5/00

(54) **DEVICE FOR OBTAINING RESPIRATORY INFORMATION OF A SUBJECT**
VORRICHTUNG ZUR ERLANGUNG VON ATEMINFORMATIONEN EINES PATIENTEN
DISPOSITIF PERMETTANT D'OBTENIR LES INFORMATIONS RESPIRATOIRES D'UNE PERSONNE

(30) Priority: 15.02.2013 EP 13155438; 15.02.2013 US 201361765085 P
(43) Date of publication of application: 23.12.2015
(73) Proprietor: Koninklijke Philips N.V., 5656 AG Eindhoven (NL)
(72) Inventor: DE HAAN, Gerard, NL-5656 AE Eindhoven (NL); SHAN, Caifeng, NL-5656 AE Eindhoven (NL); HOU, Jingqi, NL-5656 AE Eindhoven (NL)
(74) Representative: Philips Intellectual Property & Standards
(86) International application number: PCT/EP2014/052253
(87) International publication number: WO 2014/124855

(56) References cited:
- EP-A1- 2 298 176
- WO-A1-2011/088227
- JP-A- 2012 239 661
- US-A1- 2005 288 600
- US-A1- 2011 251 493
- PRATIK SAHINDRAKAR: "Improving Motion Robustness of Contact-less Monitoring of Heart Rate Using Video Analysis", 24 August 2011 (2011-08-24), Eindhoven, NL, XP055051521, Retrieved from the Internet <URL:http://alexandria.tue.nl/extra1/afstversl/wsk-i/sahindrakar2011.pdf> [retrieved on 20130129]

## Description

### FIELD OF THE INVENTION

The present invention relates to a device, method and system for obtaining respiratory information of a subject.

### BACKGROUND OF THE INVENTION

Vital signs of a person, for example the heart rate (HR) or respiratory information such as the respiratory rate (RR), can serve as a powerful predictor of serious medical events. For this reason the respiratory rate is often monitored online in intensive care units or in daily spot checks in the general ward of a hospital. The respiratory rate is one of the most important vital signs but it is still difficult to measure without body contact. In present intensive care units, thorax impedance plethysmography or the respiratory inductive plethysmography are still the methods of choice, wherein typically two breathing bands are used in order to distinguish thorax and abdominal breathing motion of a person. However, these methods are uncomfortable and unpleasant for the patient being observed.

Recently camera-based respiratory signal monitoring has been developed. With this technology, human respiration can be monitored unobtrusively from a distance with a video camera, which has advantages over traditional on-body sensor (e.g., ECG or stretch bands) based solutions. The contactless measurement is realized by analysing the video of a subject's chest (or belly) area to measure the periodic respiration motion.

WO 2012/140531 A1 discloses a respiratory motion detection apparatus for detecting the respiratory motion of a person. This detection apparatus detects electromagnetic radiation emitted and/or reflected by a person wherein this electromagnetic radiation comprises a continuous or discrete characteristic motion signal related to the respiratory rate of the person and other motion artifacts related to the movement of the person or related to ambient conditions. This apparatus increases the reliability of the respiratory rate measurement by taking into account data processing means adapted to separate the respiratory rate signal from overall disturbances by taking into account a predefined frequency band, common predefined direction or an expected amplitude band and/or amplitude profile to distinguish the different signals.

Such non-invasive respiratory rate measurements can be accomplished optically by use of a stationary video camera. A video camera captures the breathing movements of a patient's chest in a stream of images. The breathing movements lead to a temporal modulation of certain image features, wherein the frequency of the modulation corresponds to the respiratory rate of the patient monitored. Examples of such image features are the average amplitude in a spatial region of interest located around the patient's chest, or the location of the maximum of the spatial cross correlation of the region of interest in subsequent images. The quality and the reliability of the obtained vital sign information are largely influenced by the quality of the input image data influenced by an appropriate selection of the image contrast and the selected region of interest.

Since it is based on detecting subtle respiration motion in the chest/belly area, camera-based respiration monitoring is sensitive to subject's non-respiratory motion; any non-breathing motion observed in the chest/belly area could introduce measurement errors. Document US2011/251493 A1 discloses a device for obtaining respiratory information of a subject according to the prior art.

### SUMMARY OF THE INVENTION

It is an object of the present invention to provide a device, method and system for obtaining respiratory information of a subject with a higher accuracy and robustness, in particular with respect to non-breathing motion of the subject.

In a first aspect of the present invention a device for obtaining respiratory information of a subject is presented that comprises
- a motion signal computing unit for computing a number M of motion signals for a plurality of pixels and/or groups of pixels of at least a region of interest for a number N of image frames of a subject,
- a transformation unit for computing, for some or all motion signals, a number of source signals representing independent motions within said images by applying a transformation to the respective motion signals to obtain source signals representing independent motions within said image frames, and
- a selection unit for selecting a source signal from among said computed source signals representing respiration of said subject by examining one or more properties of said source signals for some or all of said computed source signals.

In further aspects of the present invention a corresponding method as well as a system for obtaining respiratory information of a subject are presented, said system comprising an imaging unit for obtaining a number N of image frames of a subject, and a device as disclosed herein for obtaining respiratory information of the subject by use of said obtained N images frames of the subject.

In yet further aspects of the present invention, there are provided a computer program which comprises program code means for causing a computer to perform the steps of the processing method when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device and as defined in the dependent claims.

The present invention is based on the idea to determine the respiratory information, in particular the respiratory rate, from a number of images (e.g. a video stream or an image sequence obtained by a camera), by several processing steps. First, motion signals, e.g. in the form of a motion vector field, are pixelwise and/or blockwise (i.e. for local regions) computed for some or all of the images, at least within a region of interest (e.g. the chest or belly area of the subject). To said motion signals a transformation, e.g. a blind signal separation (also called blind source separation), is applied to obtain a set of separated source signals that represent independent motions within said images. Finally, from said separated source signals the source signal representing respiration is selected by examining one or more properties of said source signals, for instance based on the correlation of the separated source signals with the original motion signals and/or the frequency information of the separated source signals. In this way, non-breathing related motion can be excluded and a more reliable and accurate respiration information can be obtained, for instance when unobtrusively monitoring a neonate that often shows non-breathing related motion.

In a preferred embodiment said transformation unit is configured to compute, for P of said M motion signals, a number Q of source signals representing independent motions within said images, wherein 2 ≤ P ≤ M and 2 ≤ Q ≤ P, by applying a transformation to the respective P motion signals to obtain said Q source signals representing independent motions within said N image frames. Thus, it is generally possible that less (Q) source signals than the P motion signals are calculated. Particularly with principal component analysis as transformation it may be advantageous to stop searching for further components once the variance drops below a level assumed to be too small to be respiration.

According to a preferred embodiment said selection unit is configured to examine, for some or all of said Q source signals, the eigenvalues, the variance, the frequency, and/or the correlation of the source signal with the corresponding motion signal and/or a spatial correlation. Thus, one or more options exist for selecting the right source signal that represents the respiration motion.

In an embodiment said transformation unit is configured to apply a blind signal separation to the respective motion signals. Such a blind signal separation is a useful algorithm to separate the observed mixed motion signals into different separated source signals.

Preferably, said transformation unit is configured to compute a said number of source signals by applying a principal component analysis (PCA) and/or an independent component analysis (ICA) to the respective motion signals to obtain the source signals of length N and a corresponding eigenvalues or variances. These eigenvalues measure the variance of the original motion signal data in the direction of the corresponding source signals, i.e. principal components. These analyses are useful tools for implementing the desired transformation.

With PCA and ICA the source signals are linear combinations of the P motion signals. In an embodiment the selection is based on weighting coefficients of the combination (e.g. the strongest weight is given to an area believed to be likely the chest/abdominal area). It shall be understood that these weighting coefficients are "parameters of the source signal" in the context of the present invention.

Further, said transformation unit is configured to obtain said number of source signals of length N with corresponding variances of the data in the direction of the source signals. In general, the variance of the original data in the direction of the independent signal is desired. In case of ICA the variance of the data in the direction of the coefficient vectors from which the independent components are built are preferably used. In case of PCA said number of source signals of length N is obtained with corresponding variances of the data in the direction of the principal components.

As explained above various options for selecting the source signal that represents the respiration signal exist. Often a combination of these various options will be used to get a reliable selection.

According to one embodiment said selection unit is configured to select a source signal from among said source signals by use of the eigenvalues or variances and selecting a source signal having an eigenvalue or variance that is larger than a minimum threshold and smaller than a maximum threshold for the eigenvalue. These thresholds can be empirically determined by checking the reasonable variance of the expected respiration motion in the image frames. They determine the likely frame-to-frame displacement range for a breathing subject. This will generally depend on the optics and the distance of camera to subject, but may be fixed if the range is chosen not too restrictive.

According to another embodiment said selection unit is configured to select a source signal from among said source signals by use of the dominant frequency of said source signals, wherein the source signal is selected having a dominant frequency component within a predetermined frequency range including an expected respiration rate. According to still another embodiment said selection unit is configured to select a source signal from among said source signals by use of the correlation of the source signal with the motion signals and to select the source signal having the highest correlation with motions in the chest or belly area of the subject. Finally, according to an embodiment said selection unit is configured to select a source signal from among said source signals by use of a spatial correlation and to select a source signal having the largest spatially consistent area within the image frames.

Also for the motion signal computation various options exist. In one embodiment said motion signal computing unit is configured to compute a dense or sparse motion vector field comprising said number M of motion signals. In another embodiment said motion signal computing unit is configured to process said motion vector field by down-sampling, grouping, averaging or non-linear combining of motion signals, in particular to save computation costs.

### BRIEF DESCRIPTION OF THE DRAWINGS

These and other aspects of the invention will be apparent from and elucidated with reference to the embodiment(s) described hereinafter. In the following drawings
Fig. 1 shows a diagram depicting the motion of a baby over time,
Fig. 2 shows a diagram of a respiration signal obtained by a known method compared to a reference signal,
Fig. 3 shows diagram of a device and system according to the present invention,
Fig. 4 shows a diagram of a motion vector field,
Fig. 5 shows a diagram showing several source signals,
Fig. 6 shows a diagram of the power spectrum of the source signals shown in Fig. 5, and
Fig. 7 shows a diagram of a respiration signal obtained by a proposed method compared to a reference signal.

### DETAILED DESCRIPTION OF THE INVENTION

As mentioned above the detection of respiration information is based on detecting subtle respiration motion of a body portion of the subject (generally a person, but also an animal) that shows motion caused by respiration, in particular of the chest and/or belly area. The best locations typically contain edge information (for reliable motion estimation) and move due to breathing which typically implies they are connected to the chest or abdominal region (but this can be a blanket covering a neonate, or a shoulder, or a clear detail on the sweater of an adult). Less likely areas are limbs which tend to move independently from the respiratory rate, or parts of the bedding not in mechanical contact with the chest or belly region.

Such unobtrusive, image-based respiration monitoring is sensitive to subject's non-respiratory motion, i.e. any non-breathing motion observed in the respective body portion (e.g. chest and/or belly area) potentially introduces measurement errors.

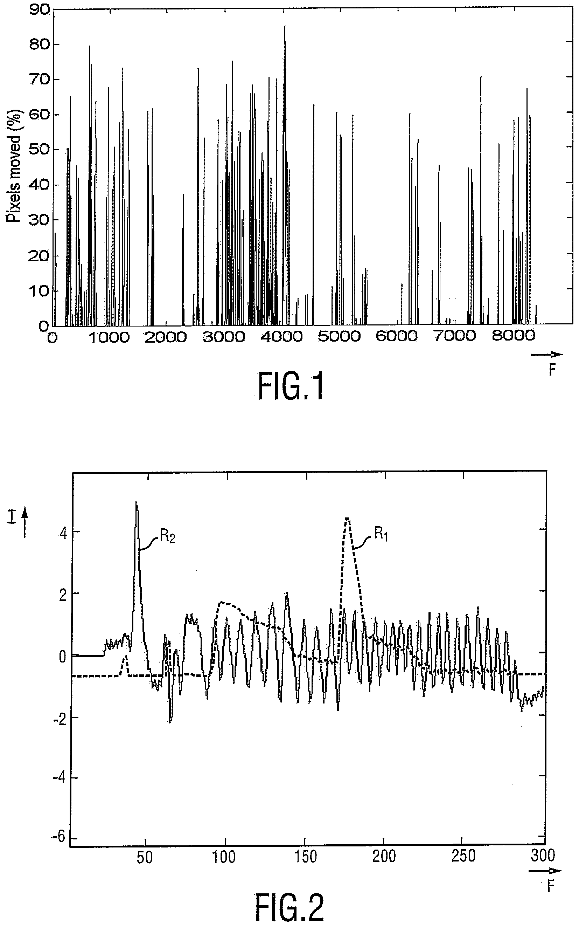

One main use scenario is contactless respiration monitoring of newborn infants at a neonatal ICU (NICU; Neonatal Intensive Care Unit). As illustrated in the signal diagram Fig. 1 depicting the motion (i.e. the percentage of pixels moved) of a baby over time (i.e. over the frame number F), a baby often has body movements when he/she is awake. The infant's non-breathing movements make the respiratory signal measurement noisy or inaccurate.

Fig. 2 shows a signal diagram of an example of current respiration monitoring when the infant's body moves. In particular, the intensity I of a measured respiration signal R1 obtained from images according to a known method and of a reference respiration signal R2 obtained by a conventional respiration rate detector (e.g. a wristband type sensor) or any other appropriate measurement equipment (in this example an ECG sensor) are compared over time (i.e. over the frame number F). As can be seen, the measured respiration signal R1 is not accurate.

Image-based (or camera-based) respiration monitoring is based on detecting the breathing motion particularly in the chest/belly area. In practice, besides respiration, other non-breathing motion the subject has (e.g. body movement) and noise also cause motion in the chest/belly area. Therefore, the observed motion signals are actually a mixture of breathing motion, non-breathing motion and noise, e.g. due to estimation errors. It is assumed that these sources are uncorrelated. According to the present invention, it is proposed to apply a transformation (e.g. a blind signal (source) separation technique, such as PCA (Principal Component Analysis) or ICA (Independent Component Analysis)) to separate the observed mixed motion signal (showing different contribution from different motion and noise) into different sources, and then select the source signal that represents respiration.

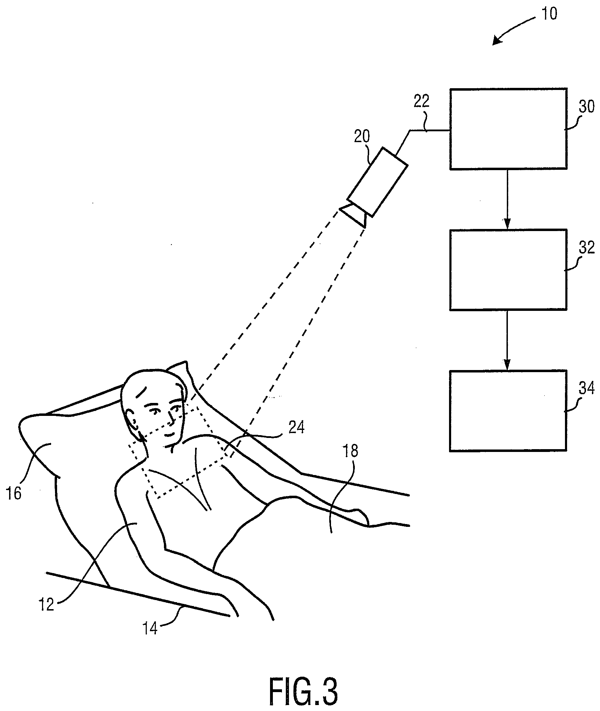

Fig. 3 shows a first exemplary embodiment of a device 10 for obtaining respiratory information of a subject 12 according to the present invention. The subject 12 lies in a bed 14, wherein the head of the subject 12 is located on a pillow 16 and the subject 12 is covered with a blanket 18. The device 10 comprises an imaging unit 20 (e.g. a video camera) for acquiring a set of image data 22 (i.e. an image sequence or video data comprising a number of image frames) detected from a body portion 24 of the subject 12 showing a motion caused by respiration, in particular from the chest or belly area. The device 10 together with the imaging unit 20 form a system 1 as proposed according to the present invention.

In general, the device 10 comprises a motion signal computing unit 30 for computing a number M of motion signals for a plurality of pixels and/or groups of pixels of at least a region of interest for a number N of image frames of a subject. Further, a transformation unit 32 is provided for computing, for some or all motion signals, a number of source signals representing independent motions within said images by applying a transformation to the motion signals to obtain source signals representing independent motions within said image frames. Finally, a selection unit 34 is provided for selecting a source signal from among said computed source signals representing respiration of said subject by examining one or more properties of said source signals for some or all of said computed source signals.

The motion signal computing unit 30, the transformation unit 32 and the selection unit 34 can be implemented by separate elements (e.g. processors or software functions), but can also be represented and implemented by a common processing apparatus or computer. Embodiment of the various units will be explained in more detail in the following.

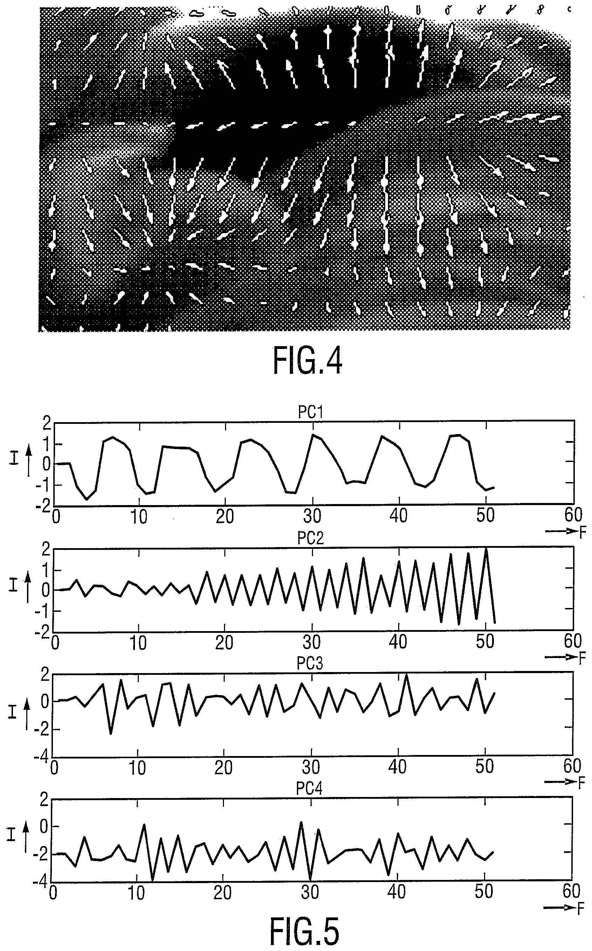

For the input images (e.g. video stream or image sequence) acquired by the imaging unit 20 (e.g. a RGB camera, infrared camera, etc.), a motion vector field is first calculated in an embodiment of the motion signal computing unit 30. Many options for motion estimation, both sparse and dense, are possible and useful. For example, an optical flow algorithm as described in Gautama, T. and Van Hulle, M.M., A Phase-based Approach to the Estimation of the Optical Flow Field Using Spatial Filtering, IEEE Trans. Neural Networks, Vol. 13(5), 2002, pp. 1127-1136, can be applied to obtain a dense motion vector field as e.g. shown in Fig. 4. Alternatively, a block-matching motion estimation algorithm as described in G. de Haan, P.W.A.C Biezen, H. Huijgen, and O.A. Ojo, True Motion Estimation with 3-D Recursive Search Block-Matching, IEEE Trans. Circuits and Systems for Video Technology, Vol. 3, 1993, pp. 368-388, or a segment-based motion estimation can be applied to obtain a motion vector per block/group/segment of pixels. Finally, Lucas-Kanade (KLT) feature tracker (or similar algorithms) can be adapted and used to find correspondence of local feature points and generate a sparse motion vector field. It is also possible and efficient to calculate motion only for so-called feature-points, e.g. detected with a Harris detector, which leads to a sparse vector field (not available for all locations) that can be input to the further algorithm.

Instead of computing motion vectors on the whole image frame, a region of interest (ROI) can be selected manually or automatically for motion vector calculation. Further, to reduce the computational cost, the dense or block-based calculated motion vector field can also be down-sampled before further processing. Also a segment-based or sparse vector field can be pre-processed to (further) reduce the number of motion vectors provided to subsequent processing. This pre-processing may involve down-sampling or grouping, and may involve non-linear combining of motion vectors using median filters, trimmed mean filters, etc.

Given the computed motion vectors, the motion signal for a plurality or each local region in the ROI is calculated, based on motion vectors inside the region. The local region can be a pixel (e.g. after down-sampling mentioned above) or a number of pixels (e.g., 3x3 pixels). The motion signal can be the median of mean of motion vectors inside the region. The motion signal at each position over N frames (e.g., N=50) is one data sample of length N. Assuming there are M points (or regions) in the ROI, a data matrix of M*N is obtained for further processing.

In an embodiment of the transformation unit 32 a blind signal separation algorithm as generally known in the art (for example, described in Cardoso, J.-F., Blind signal separation: statistical principles, Proceedings of the IEEE, 86(10), Oct 1998, pp. 2009 - 2025) (e.g., PCA) or a combination of them (e.g., PCA and ICA) is applied to the data matrix of motion signals, resulting in a set of separated source signals.

In one embodiment, PCA is adopted. The input data (MxN) for the PCA, obtained from the above explained embodiment of the motion signal computing unit 30, represents the motion of M regions over N frames in the sequence of image frames. Each of these M regions gives a motion signal with length N. By applying PCA to the MxN data matrix, a number of eigenvectors (of length M) is obtained with corresponding eigenvalues. An eigenvector contains the weights given to each of these M signals to provide a weighted average motion signal (i.e. source signal, also called principal component). The signals corresponding to individual eigenvectors are orthogonal, i.e. their covariance equals zero. In yet other words, they represent independent motions in the video. One of these is expected to be the respiration motion, which shall be found amidst distracting motion components in the sequence of image frames. On the other hand, the eigenvalues represent the variance of the original data in the direction of the corresponding source signal (principal component).

Generally, for M motion signals a number Q of source signals representing independent motions within said images (wherein 2 ≤ P ≤ M and 2 ≤ Q ≤ P) are computed in the transformation unit. The maximum number of eigenvectors equals the number of regions M. In practical situations, however, only a smaller number Q (e.g. 10 or 20 eigenvectors) with the highest eigenvalue may be used. In an embodiment a selection of the eigenvectors is used that have an eigenvalue in a reasonable range (for the expected breathing motion). For instance, in an embodiment a few hundred regions (e.g., M=200) may be considered in the ROI. The minimum number of regions should not be too low (e.g. not lower than 3).

In an embodiment of the selection unit 34, given a set of separated source signals (as exemplarily shown in Fig. 5 depicting the intensity I of four source signals over frame number F), the source signal representing respiration (i.e., providing the largest SNR for breathing motion) is selected. By examining the eigenvalue, the principal components with too large or too small eigenvalues (representing large body motion or noises) are discarded. Fig. 5 shows the remaining principal components obtained for an example video segment.

In particular, the four signals shown in Fig. 5 are the resulting independent motion signals obtained by multiplying the eigenvectors with the motion signals from the M regions. They are different linear combinations of the motion signals from the M regions in the ROI.

A further (alternative or additional) selection may use the frequency information of the separated source signals and/or the correlation of the separated source signals with the original motion signals, as will be explained in the following.

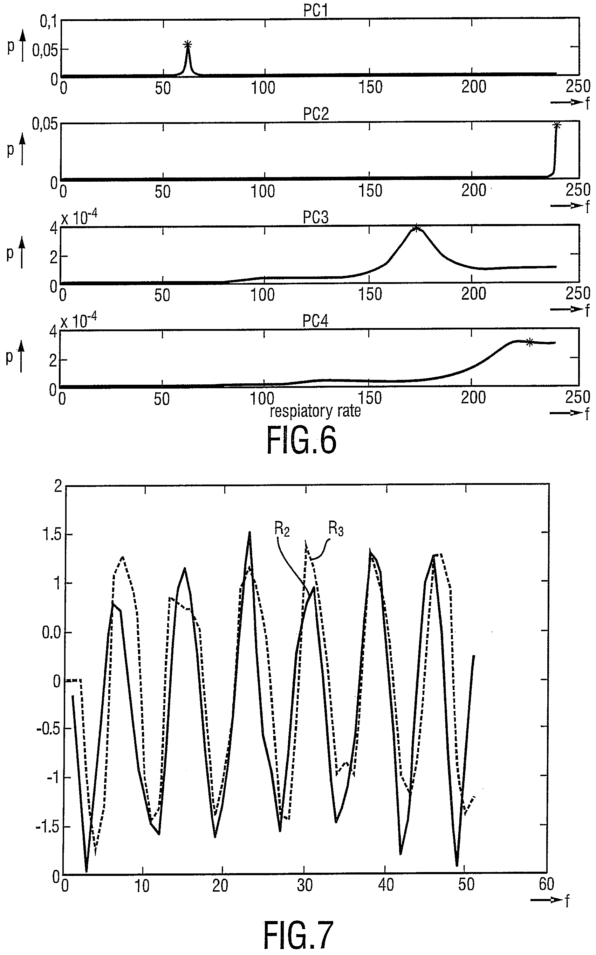

The respiration motion has certain frequency, for example, for neonates, this can be [0.5, 1.5] Hz (i.e., [30, 90] bpm). Therefore, the source signal representing breathing is supposed to have clear peak(s) in this frequency range. Fig. 6 plots the power spectrum (power p over frequency f) of each signal source shown in Fig. 5. Obviously the first one can be selected, which represents respiration very well, as shown in Fig. 7 showing the intensity of the selected source signal R3 compared to the reference signal R2 over frame number F.

According to another embodiment of the selection unit 34 the correlation of some or each source signal with the original motion signals in the input frames is determined and examined. The breathing motion is supposed to have high correlation in the chest/belly area. In practice, the chest/belly area may be known (e.g., automatically detected by used of image recognition or manually decided). By comparing the correlation of some or each source signal in the chest/belly area, the source signal representing respiration can be selected.

According to another embodiment of the selection unit 34 spatial correlations are analysed and a large consistent segment is looked for. The assumption here is that the respiration motion is found in a spatially consistent area in the image, the position of which is roughly known, while the other independent components may occur in more scattered areas and or areas where it is unlikely that breathing is present.

In summary, comparing the results of the proposed device and method (as e.g. shown in Fig. 7) with the results of known algorithms (as shown in Fig. 2) on a few video segments (when the baby has non-breathing motion) it is evident that the proposed device and method provide a more accurate and robust respiratory signal measurement.

The present invention can be used for camera-based respiration measurement, using monochrome or color cameras and visible or infrared illumination, and is relevant for many applications including patient monitoring (including neonate monitoring), home healthcare, sleep monitoring, sports (monitoring of a person during an exercise), etc.

The invention is defined by the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for obtaining respiratory information of a subject, comprising:
- a motion signal computing unit (30) for estimating motion to obtain motion vectors and computing a dense or sparse motion vector field comprising a number M of motion signals from the obtained motion vectors for a plurality of pixels and/or groups of pixels of at least a region of interest for a number N of image frames of a subject, a motion signal reflecting the estimated motion of a part of the region of interest corresponding to the pixel or group of pixels for which the motion signal is computed,
- a transformation unit (32) for computing, for some or all M motion signals, a number of source signals representing independent motions within said images by applying a transformation to the respective motion signals to obtain source signals representing independent motions within said N image frames, and
- a selection unit (34) for selecting a source signal from among said computed source signals representing respiration of said subject by examining one or more properties of said source signals for some or all of said computed source signals.

2. Device as claimed in claim 1,
wherein said transformation unit (32) is configured to compute, for P of said M motion signals, a number Q of source signals representing independent motions within said images,
wherein 2 ≤ P ≤ M and 2 ≤ Q ≤ P, by applying a transformation to the respective P motion signals to obtain said Q source signals representing independent motions within said N image frames.

3. Device as claimed in claim 2,
wherein said selection unit (34) is configured to examine, for some or all of said Q source signals, at least one of the eigenvalues, the variance, the frequency, the correlation of a source signal with the corresponding motion signal, and a spatial correlation.

4. Device as claimed in claim 1,
wherein said transformation unit (32) is configured to apply a blind signal separation to the respective P motion signals.

5. Device as claimed in claim 1,
wherein said transformation unit (32) is configured to compute said number of source signals by applying a principal component analysis and/or an independent component analysis to the respective motion signals to obtain the source signals of length N and corresponding eigenvalues or variances.

6. Device as claimed in claim 5,
wherein said transformation unit (32) is configured to obtain said number of source signals of length N with corresponding variances of the data in the direction of the source signals.

7. Device as claimed in claim 1,
wherein said selection unit (34) is configured to select a source signal from among said source signals by use of the eigenvalues or variances, and selecting a source signal having an eigenvalue or variance that is larger than a minimum threshold and smaller than a maximum threshold for the eigenvalue.

8. Device as claimed in claim 1,
wherein said selection unit (34) is configured to select a source signal from among said source signals by use of the dominant frequency of said source signals, wherein the source signal is selected having a dominant frequency component within a predetermined frequency range including an expected respiration rate.

9. Device as claimed in claim 1,
wherein said selection unit (34) is configured to select a source signal from among said source signals by use of the correlation of the source signal with the motion signals and to select the source signal having the highest correlation with motions in the chest or belly area of the subject.

10. Device as claimed in claim 1,
wherein said selection unit (34) is configured to select a source signal from among said source signals by use of a spatial correlation and to select a source signal from the largest spatially consistent area within the image frames.

11. Device as claimed in claim 1,
wherein said motion signal computing unit (30) is configured to process said motion vector field by down-sampling, grouping, averaging or non-linear combining of motion signals.

12. System for obtaining respiratory information of a subject, comprising:
- an imaging unit (20) for obtaining a number N of image frames of a subject, and
- a device as claimed in any one of claims 1 to 11 for obtaining respiratory information of the subject by use of said obtained N image frames of the subject.

13. Computer-implemented method for obtaining respiratory information of a subject, comprising:
- estimating motion to obtain motion vectors and computing a dense or sparse motion vector field comprising a number M of motion signals from the obtained motion vectors for a plurality of pixels and/or groups of pixels of at least a region of interest for a number N of image frames of a subject, a motion signal reflecting the estimated motion of a part of the region of interest corresponding to the pixel or group of pixels for which the motion signal is computed,
- computing, for some or all M motion signals, a number of source signals representing independent motions within said images by applying a transformation to the respective motion signals to obtain source signals representing independent motions within said N image frames, and
- selecting a source signal from among said computed source signals representing respiration of said subject by examining one or more properties of said source signals for some or all of said computed source signals.

14. Computer program comprising program code means for causing a computer to carry out the steps of the method as claimed in claim 13 when said computer program is carried out on the computer.

## Patentansprüche

1. Vorrichtung zum Erhalten von Atmungsinformationen eines Subjekts, umfassend:
- eine Bewegungssignal-Berechnungseinheit (30) zum Schätzen von Bewegung, um Bewegungsvektoren zu erhalten, und Berechnen eines dichten oder spärlichen Bewegungsvektorfeldes, das eine Anzahl M von Bewegungssignalen umfasst, aus den erhaltenen Bewegungsvektoren für eine Vielzahl von Pixeln und/oder Pixelgruppen mindestens eines Bereichs von Interesse für eine Anzahl N von Einzelbildern eines Subjekts, wobei ein Bewegungssignal die geschätzte Bewegung eines Teils des Bereichs von Interesse, der dem Pixel oder der Pixelgruppe entspricht, für das/die das Bewegungssignal berechnet wird, widerspiegelt,
- eine Transformationseinheit (32) zum Berechnen, für einige oder alle M Bewegungssignale, einer Anzahl von Quellensignalen, die unabhängige Bewegungen innerhalb der Bilder darstellen, durch Anwenden einer Transformation auf die jeweiligen Bewegungssignale, um Quellensignale zu erhalten, die unabhängige Bewegungen innerhalb der N Einzelbilder darstellen, und
- eine Auswahleinheit (34) zum Auswählen eines Quellensignals aus den berechneten Quellensignalen, das die Atmung des Subjekts darstellt, durch Untersuchen einer oder mehrerer Eigenschaften der Quellensignale für einige oder alle der berechneten Quellensignale.

2. Vorrichtung nach Anspruch 1,
wobei die Transformationseinheit (32) dazu konfiguriert ist, für P der M Bewegungssignale eine Anzahl Q von Quellensignalen, die unabhängige Bewegungen innerhalb der Bilder darstellen, wobei 2 ≤ P ≤ M und 2 ≤ Q ≤ P, zu berechnen durch Anwenden einer Transformation auf die jeweiligen P Bewegungssignale, um die Q Quellensignale zu erhalten, die unabhängige Bewegungen innerhalb der N Einzelbilder darstellen.

3. Vorrichtung nach Anspruch 2,
wobei die Auswahleinheit (34) dazu konfiguriert ist, für einige oder alle der Q Quellensignale mindestens eines der Eigenwerte, der Varianz, der Frequenz, der Korrelation eines Quellensignals mit dem entsprechenden Bewegungssignal und einer räumlichen Korrelation zu untersuchen.

4. Vorrichtung nach Anspruch 1,
wobei die Transformationseinheit (32) dazu konfiguriert ist, eine blinde Signaltrennung auf die jeweiligen P Bewegungssignale anzuwenden.

5. Vorrichtung nach Anspruch 1,
wobei die Transformationseinheit (32) dazu konfiguriert ist, die Anzahl von Quellensignalen durch Anwenden einer Hauptkomponentenanalyse und/oder einer unabhängigen Komponentenanalyse auf die jeweiligen Bewegungssignale zu berechnen, um die Quellensignale der Länge N und entsprechende Eigenwerte oder Varianzen zu erhalten.

6. Vorrichtung nach Anspruch 5,
wobei die Transformationseinheit (32) dazu konfiguriert ist, die Anzahl von Quellensignalen der Länge N mit entsprechenden Varianzen der Daten in Richtung der Quellensignale zu erhalten.

7. Vorrichtung nach Anspruch 1,
wobei die Auswahleinheit (34) dazu konfiguriert ist, ein Quellensignal aus den Quellensignalen durch Verwendung der Eigenwerte oder Varianzen auszuwählen, und ein Quellensignal auszuwählen, das einen Eigenwert oder eine Varianz aufweist, der/die größer als eine Mindestschwelle und kleiner als ein Höchstschwelle für den Eigenwert ist.

8. Vorrichtung nach Anspruch 1,
wobei die Auswahleinheit (34) dazu konfiguriert ist, ein Quellensignal aus den Quellensignalen durch Verwendung der dominanten Frequenz der Quellensignale auszuwählen, wobei das Quellensignal ausgewählt wird, das eine dominante Frequenzkomponente innerhalb eines vorbestimmten Frequenzbereichs, der eine erwartete Atmungsrate einschließt, aufweist.

9. Vorrichtung nach Anspruch 1,
wobei die Auswahleinheit (34) dazu konfiguriert ist, ein Quellensignal aus den Quellensignalen durch Verwendung der Korrelation des Quellensignals mit den Bewegungssignalen auszuwählen, und das Quellensignal, das die höchste Korrelation mit Bewegungen im Brust- oder Bauchbereich des Subjekts aufweist, auszuwählen.

10. Vorrichtung nach Anspruch 1,
wobei die Auswahleinheit (34) dazu konfiguriert ist, ein Quellensignal aus den Quellensignalen durch Verwendung einer räumlichen Korrelation auszuwählen, und ein Quellensignal aus dem größten räumlich konsistenten Bereich innerhalb der Einzelbilder auszuwählen.

11. Vorrichtung nach Anspruch 1,
wobei die Bewegungssignal-Berechnungseinheit (30) dazu konfiguriert ist, das Bewegungsvektorfeld durch Downsampling, Gruppieren, Mittelwertbildung oder nichtlineares Kombinieren von Bewegungssignalen zu verarbeiten.

12. System zum Erhalten von Atmungsinformationen eines Subjekts, umfassend:
- eine Bildgebungseinheit (20) zum Erhalten einer Anzahl N von Einzelbildern eines Subjekts, und
- eine Vorrichtung nach einem der Ansprüche 1 bis 11 zum Erhalten von Atmungsinformationen des Subjekts durch Verwendung der erhaltenen N Einzelbilder des Subjekts.

13. Computerimplementiertes Verfahren zum Erhalten von Atmungsinformationen eines Subjekts, umfassend:
- Schätzen von Bewegung, um Bewegungsvektoren zu erhalten, und Berechnen eines dichten oder spärlichen Bewegungsvektorfeldes, das eine Anzahl M von Bewegungssignalen umfasst, aus den erhaltenen Bewegungsvektoren für eine Vielzahl von Pixeln und/oder Pixelgruppen mindestens eines Bereichs von Interesse für eine Anzahl N von Einzelbildern eines Subjekts, wobei ein Bewegungssignal die geschätzte Bewegung eines Teils des Bereichs von Interesse, der dem Pixel oder der Pixelgruppe entspricht, für das/die das Bewegungssignal berechnet wird, widerspiegelt,
- Berechnen, für einige oder alle M Bewegungssignale, einer Anzahl von Quellensignalen, die unabhängige Bewegungen innerhalb der Bilder darstellen, durch Anwenden einer Transformation auf die jeweiligen Bewegungssignale, um Quellensignale zu erhalten, die unabhängige Bewegungen innerhalb der N Einzelbilder darstellen, und
- Auswählen eines Quellensignals aus den berechneten Quellensignalen, das die Atmung des Subjekts darstellt, durch Untersuchen einer oder mehrerer Eigenschaften der Quellensignale für einige oder alle der berechneten Quellensignale.

14. Computerprogramm, umfassend Programmcodemittel, um einen Computer zu veranlassen, die Schritte des Verfahrens nach Anspruch 13 auszuführen, wenn das Computerprogramm auf dem Computer ausgeführt wird.

## Revendications

1. Dispositif permettant d'obtenir les informations respiratoires d'une personne, comprenant :
- une unité de calcul de signal de mouvement (30) pour estimer le mouvement afin d'obtenir des vecteurs de mouvement et calculer un champ de vecteurs de mouvement dense ou clairsemé comprenant un nombre M de signaux de mouvement à partir des vecteurs de mouvement obtenus pour une pluralité de pixels et/ou des groupes de pixels d'au moins une région d'intérêt pour un nombre N de trames d'image d'une personne, un signal de mouvement reflétant le mouvement estimé d'une partie de la région d'intérêt correspondant au pixel ou au groupe de pixels pour lequel le signal de mouvement est calculé,
- une unité de transformation (32) pour calculer, pour certains ou tous les M signaux de mouvement, un nombre de signaux sources représentant des mouvements indépendants dans lesdites images en appliquant une transformation aux signaux de mouvement respectifs pour obtenir des signaux sources représentant des mouvements indépendants dans lesdites N trames d'image, et
- une unité de sélection (34) pour sélectionner un signal source parmi lesdits signaux sources calculés représentant la respiration de ladite personne en examinant une ou plusieurs propriétés desdits signaux sources pour certains ou tous lesdits signaux sources calculés.

2. Dispositif selon la revendication 1,
dans lequel ladite unité de transformation (32) est configurée pour calculer, pour P desdits M signaux de mouvement, un nombre Q de signaux sources représentant des mouvements indépendants dans lesdites images, dans lequel 2 ≤ P ≤ M et 2 ≤ Q ≤ P, en appliquant une transformation aux P signaux de mouvement respectifs pour obtenir lesdits Q signaux sources représentant des mouvements indépendants dans lesdites N trames d'image.

3. Dispositif selon la revendication 2,
dans lequel ladite unité de sélection (34) est configurée pour examiner, pour certains ou tous lesdits Q signaux sources, au moins l'une parmi les valeurs propres, la variance, la fréquence, la corrélation d'un signal source avec le signal de mouvement correspondant, et une corrélation spatiale.

4. Dispositif selon la revendication 1,
dans lequel ladite unité de transformation (32) est configurée pour appliquer une séparation de signal aveugle aux P signaux de mouvement respectifs.

5. Dispositif selon la revendication 1,
dans lequel ladite unité de transformation (32) est configurée pour calculer ledit nombre de signaux sources en appliquant une analyse des composantes principales et/ou une analyse des composantes indépendantes aux signaux de mouvement respectifs pour obtenir les signaux sources de longueur N et les valeurs propres ou variances correspondantes.

6. Dispositif selon la revendication 5,
dans lequel ladite unité de transformation (32) est configurée pour obtenir ledit nombre de signaux sources de longueur N avec des variances correspondantes des données dans la direction des signaux sources.

7. Dispositif selon la revendication 1,
dans lequel ladite unité de sélection (34) est configurée pour sélectionner un signal source parmi lesdits signaux sources en utilisant les valeurs propres ou les variances, et sélectionner un signal source présentant une valeur propre ou une variance qui est supérieure à un seuil minimum et inférieure à un seuil maximum pour la valeur propre.

8. Dispositif selon la revendication 1,
dans lequel ladite unité de sélection (34) est configurée pour sélectionner un signal source parmi lesdits signaux sources en utilisant la fréquence dominante desdits signaux sources, dans lequel est sélectionné le signal source présentant une composante de fréquence dominante dans une plage de fréquences prédéterminée incluant un taux de respiration attendu.

9. Dispositif selon la revendication 1,
dans lequel ladite unité de sélection (34) est configurée pour sélectionner un signal source parmi lesdits signaux sources en utilisant la corrélation du signal source avec les signaux de mouvement et pour sélectionner le signal source présentant la corrélation la plus élevée avec les mouvements dans la zone de la poitrine ou du ventre de la personne.

10. Dispositif selon la revendication 1,
dans lequel ladite unité de sélection (34) est configurée pour sélectionner un signal source parmi lesdits signaux sources en utilisant une corrélation spatiale et pour sélectionner un signal source à partir de la plus grande zone spatialement cohérente dans les trames d'image.

11. Dispositif selon la revendication 1,
dans lequel ladite unité de calcul de signal de mouvement (30) est configurée pour traiter ledit champ de vecteurs de mouvement par sous-échantillonnage, regroupement, calcul de la moyenne ou combinaison non linéaire de signaux de mouvement.

12. Système permettant d'obtenir les informations respiratoires d'une personne, comprenant :
- une unité d'imagerie (20) pour obtenir un nombre N de trames d'image d'une personne, et
- un dispositif selon l'une quelconque des revendications 1 à 11 pour obtenir des informations respiratoires de la personne en utilisant lesdites N trames d'image obtenues de la personne.

13. Procédé mis en œuvre par ordinateur permettant d'obtenir les informations respiratoires d'une personne, comprenant :
- l'estimation du mouvement afin d'obtenir des vecteurs de mouvement et le calcul d'un champ de vecteurs de mouvement dense ou clairsemé comprenant un nombre M de signaux de mouvement à partir des vecteurs de mouvement obtenus pour une pluralité de pixels et/ou des groupes de pixels d'au moins une région d'intérêt pour un nombre N de trames d'image d'une personne, un signal de mouvement reflétant le mouvement estimé d'une partie de la région d'intérêt correspondant au pixel ou au groupe de pixels pour lequel le signal de mouvement est calculé,
- le calcul, pour certains ou tous les M signaux de mouvement, d'un nombre de signaux sources représentant des mouvements indépendants dans lesdites images en appliquant une transformation aux signaux de mouvement respectifs pour obtenir des signaux sources représentant des mouvements indépendants dans lesdites N trames d'image, et
- la sélection d'un signal source parmi lesdits signaux sources calculés représentant la respiration de ladite personne en examinant une ou plusieurs propriétés desdits signaux sources pour certains ou tous lesdits signaux sources calculés.

14. Programme informatique comprenant des moyens de code de programme pour amener un ordinateur à réaliser les étapes du procédé selon la revendication 13 lorsque ledit programme informatique est exécuté sur l'ordinateur.
